(19) Europäisches Patentamt
European Patent Office
Office européen des brevets

(11) Publication number: **0 047 649**
**A2**

# (12) EUROPEAN PATENT APPLICATION

(21) Application number: **81304056.5**

(22) Date of filing: **04.09.81**

(51) Int. Cl.³: **C 09 K 15/14**, C 09 K 15/08, C 08 K 5/00, C 08 L 23/00, C 07 C 149/00, H 01 B 3/00

(30) Priority: **08.09.80 GB 8028999**

(43) Date of publication of application: **17.03.82 Bulletin 82/11**

(84) Designated Contracting States: **AT BE CH DE FR GB IT LI LU NL SE**

(71) Applicant: **THE ELECTRICITY COUNCIL, 30 Millbank, London, SW1P 4RD (GB)**

(72) Inventor: **Poldevin Le, Graham John, 108 Marshlands Road, Little Neston Merseyside, L64 OTS (GB)**

(74) Representative: **Hardisty, David Robert et al, BOULT, WADE & TENNANT 27 Furnival street, London EC4A IPQ (GB)**

(54) **Process for preparing an antioxidant, polyolefin or rubber composition including prepared antioxidant and electric cable formed therefrom.**

(57) An antioxidant for polyolefins or rubbers is prepared by reacting a polyphenol having at least one dialkyl 4-hydroxybenzyl radical with a bridging compound which is $SCl_2$, $S_2Cl_2$, formaldehyde, dichloromethane or 1,2-dichlorethane, wherein the polyphenol is a compound of the formula

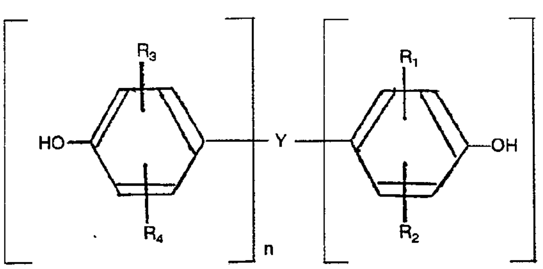

I.

wherein 'n' is at least 1, (n+m) is at least 2 and not more than 6, $R_1$ and $R_2$ are independently an alkyl group having not more than eight carbon atoms, $R_3$ and $R_4$ are independently hydrogen or the same as $R_1$ and $R_2$, and Y is a group forming a core to which the phenolic groups are attached.

EP 0 047 649 A2

0047649



PROCESS FOR PREPARING AN ANTIOXIDANT, POLYOLEFIN OR RUBBER COMPOSITION INCLUDING PREPARED ANTIOXIDANT AND ELECTRIC CABLE FORMED THEREFROM.

The invention relates to antioxidants and more particularly to antioxidants for use in polymeric compositions and also to such compositions containing the antioxidants.

It has long been known that water has a deleterious effect on polymeric compositions comprising polyolefins such as polyethylene, and this is primarily due to a loss of the antioxidants present in such compositions. The deleterious effect of water is more pronounced when the water is at an elevated temperature, e.g. greater than 60°C; it has been shown that the immersion of a polyolefin composition comprising an antioxidant in water at a temperature of 93°C. is more deleterious than the effect of circulating hot air at 105°C. on a composition placed in an oven.

A particularly important situation where aqueous solutions can be troublesome is in the case of a modern power cable having a polyethylene insulation which is at risk in those kinds of ground water containing salts. The antioxidants of the present invention are useful in insulation applications for power cables.

According to the present invention there is provided a process for preparing an antioxidant, which process comprises reacting a polyphenol having at least one dialkyl 4-hydroxybenzyl radical with a bridging compound which is $SCl_2$, $S_2Cl_2$, formaldehyde, dichlormethane or 1,2-dichloroethane, where the polyphenol is a compound of the formula

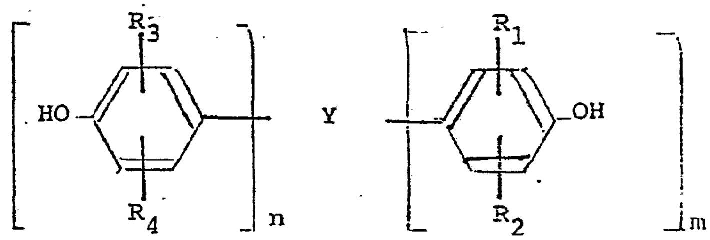

I

wherein n is at least 1 and is preferably 1 or 2, and (n + m) is from 2 to 6, $R_1$ and $R_2$ are each independently an alkyl group having up to 8 carbon atoms, the term "alkyl" as used herein including cycloalkyl groups, $R_3$ and $R_4$ are independently hydrogen or the same as $R_1$ and $R_2$ and Y is a group forming a nucleus or core to which the phenolic groups, which provide the antioxidant qualities, are attached. More specifically Y may be a bi- to hexavalent hydrocarbyl, thiohydrocarbyl, thio or dithio radical. Some specific examples of group Y are:-

$-S-$, $-CH_2-S-CH_2-$, 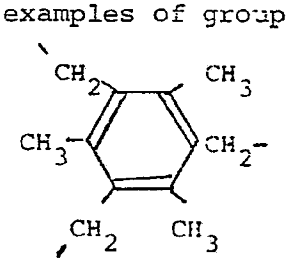 , $CH_3CHCH_2CH$.

In an alternative embodiment, the polyphenol may be a compound of the formula

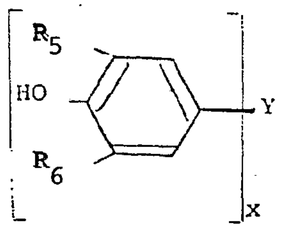

II

where $R_5$ and $R_6$ are each independently alkyl groups having up to 8 carbon atoms and Y is as defined above and x is from 2 to 6. In order to achieve the bridging of two or more molecules of initial antioxidants having a structure in accordance with formula II, de-alkylation is required to remove one or more of the hindering groups per molecule in order to free a position at which a bridging reaction can take place. This is preferably carried out using a Friedel Krafts type catalyst, such as $ZnCl_2$.

For an antioxidant of high solubility in n-octane and in polymers such as polyolefins and the like which are non-polar $R_1$ to $R_6$ each preferably contains at least 4 carbon atoms, and they may be the same or different.

The initial polyphenol to be reacted to provide the antioxidants of this invention may consist of a mixture of different antioxidants having the general formula I, and/or II, i.e. there may be a distribution

of n, m, x, Y, $R_1$, $R_2$, $R_3$, $R_4$, $R_5$ and $R_6$ in a reaction mixture from which the novel antioxidant is produced. In general the reaction to produce the present novel antioxidants involves the introduction of one or more bridging goups ortho to the hydroxyl group of the phenol. Thus a typical antioxidant according to this invention may have the general formula:-

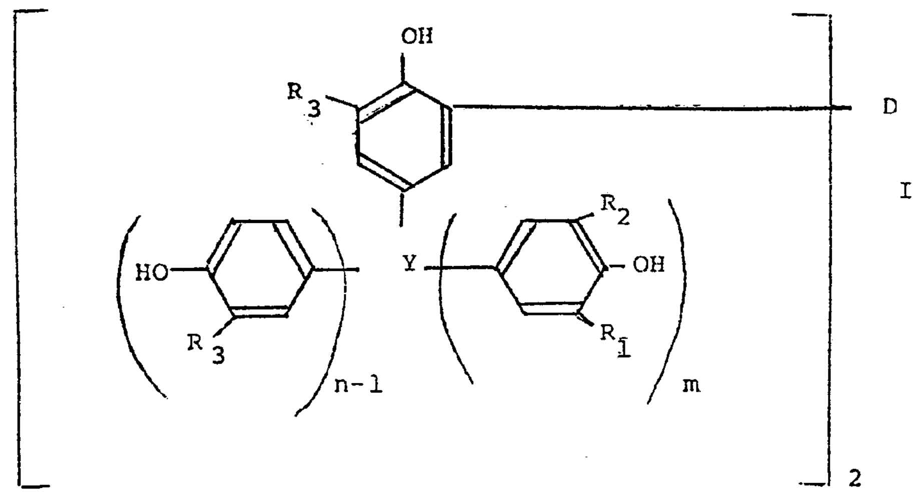

III

where the bridging group D is -S-. -S-S- or an alkylene biradical and $R_1$, $R_2$, $R_3$, Y, n and m are as defined above.

The compound of formula II is preferably an antioxidant sold under the trade name IONOX 330, or IRGONOX 1330, both of which are 1,3,5 trimethyl -2,4,6-tris (3,5-di-tert-butyl-4-Hydroxybenzyl) benzene; this compound has the formula:-

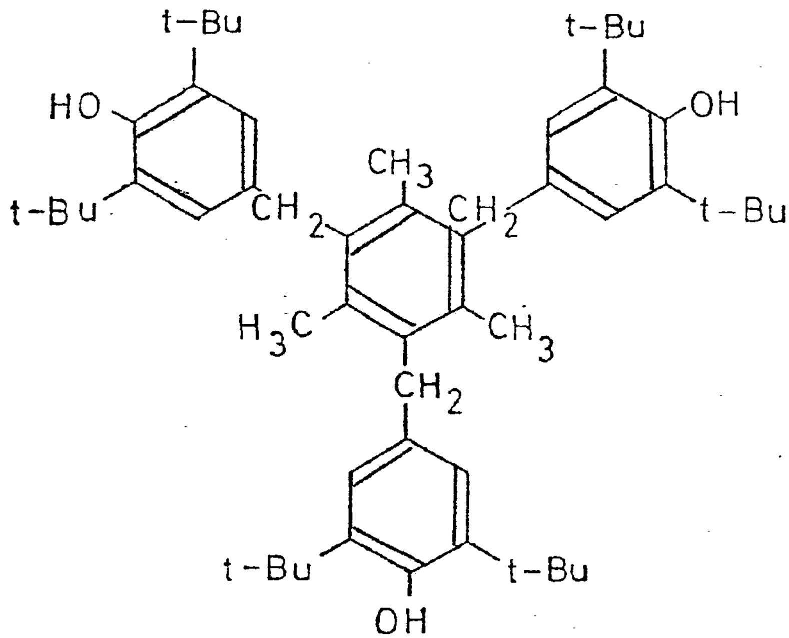

IV

As will be described in greater detail in the following Example, this compound may be subjected to a partial dealkylation of the alkylated phenol ring followed by the introduction of a thio, dithio or methylene bridge. The introduction of these bridges may be achieved, respectively, by the use of $SCl_2$, $S_2Cl_2$, formaldehyde, dichloromethane or 1,2-dichloroethane.

It is believed that the antioxidant according to this invention which is obtained by the partial dealkylation of IONOX 330 and the subsequent reaction with $S_2Cl_2$, has the following formula

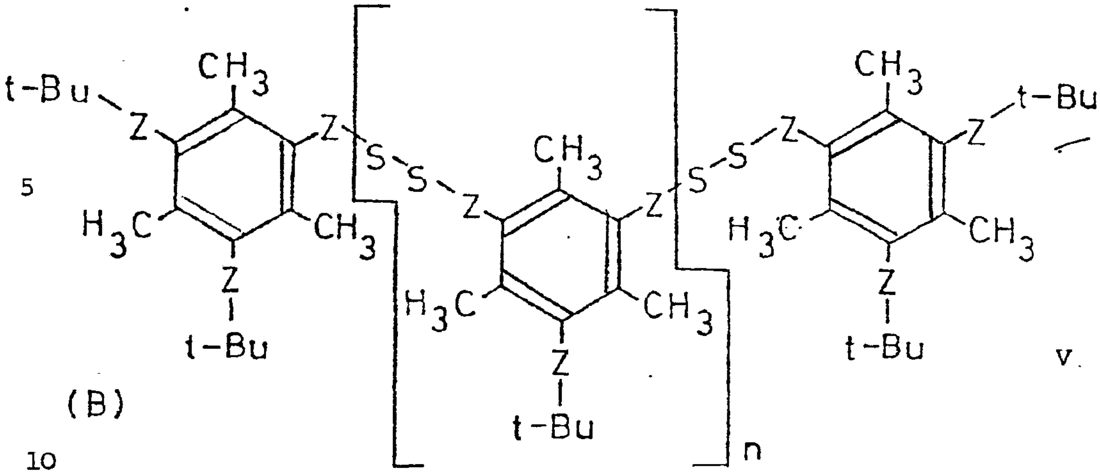

(B) V.

where Z is 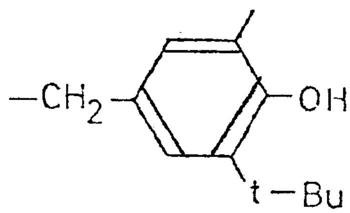

The antioxidants of the present invention are preferably soluble in n-octane to the extent of at least 5g per 100g of n-octane at 20°C.

Most commercial phenolic antioxidants are symmetrical compounds with high melting temperatures and tend to be somewhat insoluble in hydrocarbon polymers. At low temperatures such antioxidants tend to diffuse slowly to the surface or agglomerate and crystallise within the polymer. The former process is apparently accelerated by hot water, since the mixture of water and antioxidant is less compatible with the polymer than with the antioxidant alone.

The novel antioxidants according to this invention may have from 2 to 5 or more of the initial polyphenol moieties present in the molecule and these relatively higher molecular weight antioxidants are less susceptible to the difficulties referred to above. The novel antioxidants are particularly suitable for stabilizing normally solid polymers of alpha olefins, for example ethylene and propylene and their various copolymers. A wide range of polymers derived from monoolefins may be used in admixture with the present novel antioxidants to form useful polymeric compositions. The invention also includes such compositions.

It is desirable that the polymeric compositions contain a stabilizing amount of the novel antioxidants according to this invention to stabilize the composition against oxidative and/or thermal deterioration. For this purpose the antioxidant may be present in an amount of greater than 0.01% and useful loadings will comprise 0.1% or 0.2% of the antioxidant, although significantly greater amounts e.g. 1% or 5% or more may be used; the percentages are by weight.

In addition to synthetic polymers referred to above the present antioxidants may be used to stabilize other polymeric compositions including natural and synthetic rubbers.

The antioxidants and the polymer may be compounded using a Banbury mill or other means well known to those skilled in the art.

The compositions are relatively easy to blend because of the low melting point of the present antioxidants and they have good compatibility with the polymers mentioned above.

The following is a description by way of example of one method of making an antioxidant in accordance with this invention.

23.25 g (0.03 g-mole) of IONOX 330 were dissolved by gently warming and stirring, in 50 ml of dichlorethane. To this first mixture was added 0.408 g (0.003 g-mole) of anhydrous zinc chloride as catalyst. A second mixture was prepared by adding 50 cc of dichlorethane to 2.70 g (0.02 g-mole) of sulphur monochloride ($S_2Cl_2$) and stirring. The mixture of IONOX 330, catalyst and solvent was continually stirred in a flask maintained at 60°C in a thermostat. The $S_2Cl_2$/dichlorethane mixture was then added dropwise over a period of 20 minutes to the mixture in the flask. During this period the contents of the flask turned from colourless to maroon and thence to a yellowish brown. Ten minutes after the addition of the last drop the mixture in

the flask was decanted into a litre of cold distilled water and stirred for a few minutes to remove catalyst and HCl from the organic layer. The organic layer was then separated from the aqueous layer and mixed with 100 cc of xylene (mixed isomers). The new mixture of solvents and antioxidant was heated to 150°C to drive off solvent. The purpose of the xylene was to convert any unreacted chlorine in the structure ASSCl (where A is the original antioxidant minus a tertiary butyl group) to form 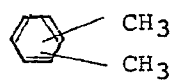 . When all solvents had been removed an amber colour glass remained. This was then crushed to form a cream coloured powder.

The stabilized compositions in accordance with this invention find particular utility in the manufacture of high voltage polymeric insulation and cable sheaths. Such cables and the like employing insulation and sheaths which comprise a stabilizing amount of one of the novel antioxidants may be used in situations where there is present environmental water, e.g. in buried insulation, or above ground where the effects of weathering will be experienced. The novel antioxidants are also particularly useful where polymeric substances of the type referred to above are to be used for hot water pipes and hot water containers.

The antioxidant resulting from the Example set out above was incorporated into a low density polyethylene supplied by Imperial Chemical Industries Limited under the designation XDB 58 at a 0.05% by weight level. The product of the Example was compared with IONOX 330, the starting material for the preparation, and the results are set forth in the following Table. The oxidation induction period was ascertained as this effectively determines the useful life of a polymeric composition, since the onset of oxidation brings deterioration in the chemical and electrical properties. At high temperatures the deterioration is rapid once the oxidation sets in. Oxidations were carried out in 150 g $l^{-1}$ sodium chloride solution at 60°C and 90°C, and in distilled water at 90°C. Solubilities in n-octane were measured at 20°C and give an idea of the relative solubilities of the antioxidants in polyethylene. Note that solubilities are very much lower in polyethylene than in n-octane because of polymer molecular weight and crystallinity effects.

TABLE 1

EFFECT OF ANTIOXIDANT PROPERTIES ON OXIDATION INDUCTION PERIOD.

| | Induction Period (Days) | | | | | |
|---|---|---|---|---|---|---|
| ANTIOXIDANT | 150 g $l^{-1}$ NaCl, 60°C | 150 g $l^{-1}$ NaCl, 90°C | Distilled Water, 90°C | Solubility in n-Octane (g/100g) | Molecular Weight | Melting Point (°C) |
| IONOX 330 | 73 | 12 | 49 | 0.52 | 775 | 239 |
| PRODUCT OF EXAMPLES | >500 | 98 | >365 | ∞ | ~1,000 | 85-95 |
| TETRAPHENOL | >500 | 31 | 140 | 0.16 | 1,168 | 114 |

Also included in Table 1 is the well known tetraphenolic antioxidant tetrakis /methylene-3-(3',5'-di-t-butyl-4'-hydroxyphenyl)propionate/ methane which is frequently used for its good resistance to hot aqueous solutions.

The results indicate that the new antioxidant is markedly superior in protecting polyethylene from oxidation in aqueous solutions, probably because of vastly superior solubility characteristics. It is significant that up to 1% of the new antioxidant can be incorporated into low density polyethylene with no blooming of antioxidant at use temperature (ambient to 80°C).

Further the procedure of the above Example may be modified to employ as the bridging compound dichloromethane or 1,2-dichloroethane; it will be understood by those skilled in the art that these bridging compounds will preferably be used with a suitable Friedel-Crafts catalyst, for example aluminium chloride. Also, the bridging compound may alternatively be $SCl_2$ or formaldehyde. Further any suitable inert solvent may be employed.

BAD ORIGINAL

CLAIMS:

1. A process for preparing an antioxidant, which process comprises reacting a polyphenol having at least one dialkyl 4-hydroxybenzyl radical with a bridging compound which is $SCl_2$, $S_2Cl_2$, formaldehyde,dichloromethane or 1,2-dichlorethane, wherein the polyphenol is a compound of the formula

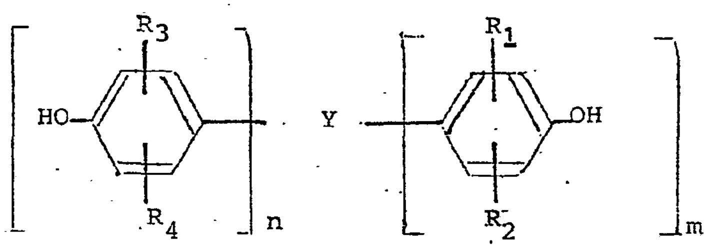

I.

wherein 'n' is at least 1, (n + m) is at least 2 and not more than 6, $R_1$ and $R_2$ are independently an alkyl group having not more than eight carbon atoms, $R_3$ and $R_4$ are independently hydrogen or the same as $R_1$ and $R_2$, and Y is a group forming a core to which the phenolic groups are attached.

2. A process as claimed in Claim 1 wherein $R_1$ $R_2$ and $R_3$ are each a t-butyl group and $R_4$ is hydrogen, and $R_1$ and $R_2$ are in the 3 and 5 positions of the 4-hydroxybenzyl radical.

3. A process as claimed in Claim 1, wherein the polyphenol is a compound of the formula

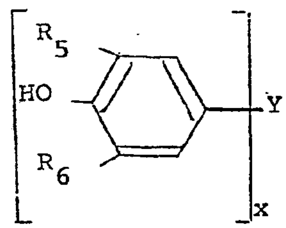

II

wherein $R_5$ and $R_6$ are each independently an alkyl group having not more than 8 carbon atoms, and X is from 2 to 6, and the reaction with the bridging compound is carried out in the presence of a dealkylating agent

4. A process as claimed in Claim 3, wherein $R_5$ and $R_6$ are each a t-butyl group.

5. A process as claimed in any one of the preceding claims, wherein Y is a bi-to hexavalent hydrocarbyl or thiohydrocarbyl group, or a thio or dithio group.

6. A process as claimed in Claim 5, wherein Y is:-

$$-S-$$

$$-CH_2-S-CH_2-$$

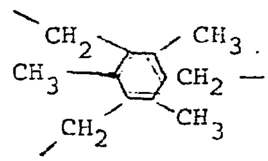

or 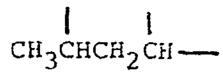

7. A process as claimed in Claim 3, wherein the polyphenol is a compound of the formula II as defined herein.

8. A polyolefin or rubber composition characterised in that there is used as an antioxidant the product of a process as defined in anyone of Claims 1 to 7.

9. A cable for conducting electricity comprising a polymeric insulation characterised in that the insulation is formed from a composition as claimed in Claim 8.

DRH/PO/EA 452